# EUROPEAN PATENT APPLICATION

(11) **EP 4 825 751 A1**
(43) Date of publication of application: **21.10.2026**
(21) Application number: 25777281.4
(22) Date of filing: 24.03.2025
(51) Int. Cl.: B65D 85/18, A61F 13/15, A61F 13/496

(54) **ACCOMMODATING BODY FOR PANTS-TYPE ABSORBENT ARTICLES**

(30) Priority: 25.03.2024 JP 2024048788
(71) Applicant: Unicharm Corporation, Ehime 799-0111 (JP)
(72) Inventor: KATO, Nobuyuki, Kanonji-shi, Kagawa 769-1602 (JP); SUZUKI, Kenta, Kanonji-shi, Kagawa 769-1602 (JP); MUKAI, Hirotomo, Kanonji-shi, Kagawa 769-1602 (JP); UTSUNOMIYA, Hideto, Kanonji-shi, Kagawa 769-1602 (JP); NARA, Yuki, Kanonji-shi, Kagawa 769-1602 (JP)
(74) Representative: Staeger & Sperling Partnerschaftsgesellschaft mbB
(86) International application number: PCT/JP2025/011451
(87) International publication number: WO 2025/205601

(57) **Abstract**

Provided is a pants-type absorbent article accommodating body (100) including a plurality of pants-type absorbent articles (1) and an accommodating member (101), wherein, in the absorbent articles (1) in an accommodated state: a vertical end portion (WA) of a waist portion (20, 30) is folded back to a crotch portion (40) side; a vertical end (WAe) of the waist portion (20, 30) does not extend beyond a vertical end (40e) of the crotch portion (40); the plurality of absorbent articles (1) are compressed in a state in which the absorbent articles (1) are stacked in the thickness direction; and in at least one of the plurality of absorbent articles (1), a distance (L1) from the end (WAe) of the waist portion (20, 30) at a central position in the left-right direction to the end (40e) of the crotch portion (40) is greater than an average thickness (T) of the crotch portion (40).

## Description

### FIELD

The present invention relates to a package for pants-type absorbent articles.

### BACKGROUND

Conventionally, absorbent articles such as disposable diapers have been known, each of which has, in a crotch portion corresponding to the wearer's crotch region, elastic members that can stretch/contract in the longitudinal direction (up-down direction) and are disposed at the center in the width direction. For example, Patent Document 1 discloses a disposable diaper that includes: a front portion and a back portion corresponding to a waist portion; an absorbent core extending from the front portion to the back portion; and elastic members disposed at the center of the absorbent core and extending in the longitudinal direction on the non-skin contact side relative to the absorbent core.

### [CITATION LIST]

### [PATENT LITERATURE]

[Patent Document 1] Japanese Patent Application Publication No. 2010-131196

### SUMMARY

### [TECHNICAL PROBLEM]

Absorbent articles such as disposable diapers described in Patent Document 1 are usually sold being stored within a containment member (storage bag, etc.) which protects absorbent articles, in a state where both lateral ends of the waist portion are folded toward the center, then folded one time or the like, and with a large compressive force applied. In other words, in order to improve shipping costs, a package, which packs a plurality of absorbent articles, tends to be able to store absorbent articles more compactly and in greater numbers by applying a high compressive force to the absorbent articles. However, if such a compressed state continues within the package, an adhesive, which had been applied to elastic members of the absorbent articles, could seep out and adhere to other adjacent articles within the containment member, or the waist portion and the crotch portion of the absorbent article could adhere to each other, causing a tear and potentially leading to the elastic members being removed.

The present invention has been made in view of the above-mentioned problems, and an aspect of the present invention is to provide a package for pants-type absorbent articles that prevents the articles from adhering to each other due to the seeping-out adhesive, in the containment member where a plurality of absorbent articles are packed in a compressed state.

### [SOLUTION TO PROBLEM]

A main aspect of the present invention of achieving the above-described aspect is a package for pants-type absorbent articles including: a plurality of pants-type absorbent articles; and a containment member that stores the plurality of pants-type absorbent articles, the pants-type absorbent article having an up-down direction, a lateral direction, and a thickness direction that are orthogonal to one another; the pants-type absorbent article including: a pair of waist portions; a crotch portion that is located in a wearer's crotch; and a central elastic member that is attached to a central portion, in the lateral direction, of the crotch portion with an adhesive, and can stretch/contract in the up-down direction, in a packaged state in which the pants-type absorbent article is stored in the containment member, an end portion, in the up-down direction, of the waist portion being folded back towards the crotch portion, an end, in the up-down direction, of the folded waist portion not extending beyond an end, in the up-down direction, of the crotch portion, the plurality of pants-type absorbent articles being compressed in a state of being stacked in the thickness direction, and in at least one of the plurality of pants-type absorbent articles, a distance, at a lateral center position, from the end of the waist portion to the end of the crotch portion being longer than an average thickness of the crotch portion. Features of the present invention other than the above will become clear by reading the description of the present specification with reference to the accompanying drawings.

### [ADVANTAGEOUS EFFECT OF THE INVENTION]

According to the present invention, it is possible to provide a package for pants-type absorbent articles that prevents the articles from adhering to each other due to the seeping-out adhesive, in the containment member where a plurality of absorbent articles are packed in a compressed state.

### BRIEF DESCRIPTION OF DRAWINGS

FIG. 1 is a schematic perspective view of the package 100 for disposable diapers.
FIG. 2 is a schematic perspective view of a diaper 1 when viewed from the front side.
FIG. 3 is a schematic plan view of the diaper 1 in an unfolded and stretched state, when viewed from the skin side of the wearer.
FIG. 4A is a schematic cross-sectional view taken along line A-A in FIG. 3, and FIG. 4B is a schematic cross-sectional view taken along line B-B in FIG. 3.
FIGS. 5A to 5C are diagrams illustrating how the diaper 1 is folded when stored.
FIG. 6 is a schematic side view of the diaper 1 in a packaged state where the diaper 1 is packed in a containment member 101.
FIG. 7 is a diagram illustrating adjacent diapers 1 in the packaged state.
FIG. 8 is a table showing the folding patterns of the diaper 1.
FIG. 9 is a diagram illustrating a modified example of a folded-back portion of the diaper 1 in the packaged state.
FIG. 10 is a diagram illustrating another modified example of the folded-back portion of the diaper 1 in the packaged state.
FIG. 11 is a diagram illustrating yet another modified example of the folded-back portion of the diaper 1.
FIG. 12 is a partial enlarged view of a front waist portion 20 in the unfolded and stretched state.

### DESCRIPTION OF EMBODIMENTS

At least the following matters become clear in the description of this specification and the accompanying drawings.

Aspect 1 is a package for pants-type absorbent articles including: a plurality of pants-type absorbent articles; and a containment member that stores the plurality of pants-type absorbent articles, the pants-type absorbent article having an up-down direction, a lateral direction, and a thickness direction that are orthogonal to one another; the pants-type absorbent article including: a pair of waist portions; a crotch portion that is located in a wearer's crotch; and a central elastic member that is attached to a central portion, in the lateral direction, of the crotch portion with an adhesive, and can stretch/contract in the up-down direction, in a packaged state in which the pants-type absorbent article is stored in the containment member, an end portion, in the up-down direction, of the waist portion being folded back towards the crotch portion, an end, in the up-down direction, of the folded waist portion not extending beyond an end, in the up-down direction, of the crotch portion, the plurality of pants-type absorbent articles being compressed in a state of being stacked in the thickness direction, and in at least one of the plurality of pants-type absorbent articles, a distance, at a lateral center position, from the end of the waist portion to the end of the crotch portion being longer than an average thickness of the crotch portion.

According to Aspect 1, by separating the end of the waist portion and the end of the crotch portion, the separating portion becomes thinner compared to cases where the absorbent article is folded such that the end of the waist portion and the end of the crotch portion coincide or are close together. This weakens the compressive force applied to the separating portion, making it less likely for the absorbent article to come into contact with another absorbent article adjacent in the thickness direction. This makes it possible to better prevent products from sticking together due to seeping out of the adhesive applied to the central elastic member, compared to cases where the ends are not separated.

Aspect 2 is a package of a pants-type absorbent article according to Aspect 1, wherein letting the distance be a length L1, and letting a distance, at a lateral center position of another pants-type absorbent article that is adjacent in the thickness direction to the pants-type absorbent article in the packaged state, from the end, in the up-down direction, of the folded waist portion to the end, in the up-down direction, of the crotch portion be a length L2, the length L2 is different from the length L1, and the length L2 is longer than at least half the length L1 or is shorter than 1.5 times the length L1.

According to Aspect 2, compared to cases where the positions of the ends of the folded waist portions of the respective pants-type absorbent articles are aligned in the containment member, the pressure is distributed in cases where the positions of the ends are different. This makes it less likely for the crotch portion where the central elastic member is located and another pants-type absorbent article to adhere to each other.

Aspect 3 is a package of a pants-type absorbent article according to Aspect 1 or 2, wherein the average thickness is 95% or less of an average thickness of the crotch portion of the pants-type absorbent article in a natural state where the pants-type absorbent article is taken out of the containment member.

According to Aspect 3, by increasing the magnitude of the compressive force applied, more pants-type absorbent articles can be stored within the containment member. Furthermore, even if the magnitude of the compressive force is increased, since the distance from the end of the folded waist portion to the end of the crotch portion is longer than the average thickness of the crotch portion, the thickness of the crotch portion becomes thinner. This makes it less likely for the crotch portion to come into contact with another pants-type absorbent article, thereby suppressing adhesion by the adhesive that has seeped out.

Aspect 4 is a package of a pants-type absorbent article according to any one of Aspects 1 to 3, wherein the central elastic member is a thread-like one.

According to Aspect 4, the amount of the adhesive for attaching the central elastic member can be reduced compared to cases of applying adhesive to the entire surface of a thicker strip-shaped rubber or sheet-shaped stretchable material (stretchable film, stretchable nonwoven fabric, etc.) and then placing it. This makes it possible to further reduce the risk that the adhesive seeps out when compressed.

Aspect 5 is a package of a pants-type absorbent article according to any one of Aspects 1 to 4, wherein the pair of waist portions are provided with a plurality of waist elastic members that stretch/contract in the lateral direction, arranged side-by-side in the up-down direction, and an amount of an adhesive per unit area of a waist elastic region where the waist elastic members are arranged is less than an amount of an adhesive per unit area of a central elastic region where the central elastic member is arranged.

According to Aspect 5, in the packaged state, the waist portions are portions that becomes thicker by being folded. By reducing the amount of the adhesive in the waist elastic members, which are arranged in such portions, the risk of the adhesive seeping out when compressed is reduced.

Aspect 6 is a package of a pants-type absorbent article according to any one of Aspects 1 to 5, wherein, in the packaged state, an upper end of the waist portion that is folded back towards the crotch portion does not overlap with the central elastic member when viewed in the thickness direction.

According to Aspect 6, when compressed in the containment member, it becomes easier to avoid the adhesion between the waist portion and the central elastic member in a compressed state due to adhesive that has seeped out.

Aspect 7 is a package of a pants-type absorbent article according to any one of Aspects 1 to 6, wherein, in the packaged state, both lateral end portions of the waist portion are folded back inward in the lateral direction.

According to Aspect 7, the portion where the both end portions of the waist portion that are folded inward in the lateral direction becomes thicker, and this thicker portion is more likely to come into contact with another absorbent article adjacent in the containment member. Because the portion where the central elastic member is located is thinner than the portion where the both end portions are folded back, even when the pants-type absorbent articles are stacked on each other, the portion where the central elastic member is located is less likely to come into contact with another absorbent article.

Aspect 8 is a package of a pants-type absorbent article according to any one of Aspects 1 to 7, wherein, in the packaged state, the pants-type absorbent article has an overlapping portion where the lateral one-side end portion and the lateral other-side end portion of the folded waist portion overlap when viewed in the thickness direction, and the overlapping portion has a portion that overlaps the central elastic member with respect to the lateral direction.

According to Aspect 8, the portion where the end portions overlap when viewed in the thickness direction becomes thicker, and therefore a difference in thickness occurs between the portion where this thickened portion and the central elastic member overlap with respect to the lateral direction, so that the portion where the central elastic member is located becomes thinner compared to the thickened portion. The aforementioned thickened portion acts as a cushion, making it less likely for the portion of the central elastic member in the containment member to be susceptible to compression and suppressing seeping out of adhesive.

Aspect 9 is a package of a pants-type absorbent article according to Aspect 7, wherein, in the packaged state, the lateral one-side end portion and the lateral other-side end portion of the folded waist portion do not overlap when viewed in the thickness direction, and in the lateral direction, when a region where the central elastic member is located is defined as a central elastic region, both ends of the waist portion are located within the central elastic region.

According to Aspect 9, since the lateral one-side end portion and the lateral other-side end portion of the folded waist portion do not overlap when viewed in the thickness direction, the waist portion is folded back with a gap in the lateral direction between the one-side end and the other-side end of the waist portion. Since, in the lateral direction, both ends (one-side end and the other-side end) of the waist portion are located within the central elastic region, the aforementioned gap can suppress the pressure on the central elastic region.

Aspect 10 is a package of a pants-type absorbent article according to Aspect 7, wherein, within the containment member, there are: a first pants-type absorbent article that has, in the packaged state, a portion where the lateral one-side end portion and the lateral other-side end portion of the folded waist portion overlap when viewed in the thickness direction; and a second pants-type absorbent article in which, in the packaged state, the lateral one-side end portion and the lateral other-side end portion of the folded waist portion do not overlap when viewed in the thickness direction.

According to Aspect 10, since the size of the room that can accommodate multiple pants-type absorbent articles as the package is fixed, the mixing of the first pants-type absorbent article with the second pants-type absorbent article distributes the areas of high pressure, making it less likely for adhesive to transfer as a whole.

Aspect 11 is a package of a pants-type absorbent article according to any one of Aspects 1 to 10, wherein, in the crotch portion in the packaged state, when a side in the thickness direction where the folded waist portion exists is defined as a fold-back inner side, and the opposite side is defined as a fold-back outer side, a length, in the up-down direction, of the central elastic member is longer on the fold-back inner side than on the fold-back outer side.

According to Aspect 11, due to the shorter length of the central elastic member on the fold-back outer side, it is possible to reduce the risk that the central elastic member and another pants-type absorbent article adjacent to the fold-back outer side adhere to each other.

Aspect 12 is a package of a pants-type absorbent article according to any one of Aspects 1 to 10, wherein, in the crotch portion in the packaged state, when a side in the thickness direction where the folded waist portion exists is defined as a fold-back inner side, and the opposite side is defined as a fold-back outer side, a length, in the up-down direction, of the central elastic member is longer on the fold-back outer side than on the fold-back inner side.

According to Aspect 12, due to the shorter length of the central elastic member on the fold-back inner side, it is possible to reduce the risk that the central elastic member and another pants-type absorbent article adjacent to the fold-back inner side adhere to each other.

Aspect 13 is a package of a pants-type absorbent article according to any one of Aspects 1 to 12, wherein, in the packaged state, when a portion of the waist portion that is folded back towards the crotch portion is defined as a folded-back portion, the folded-back portion has a portion that overlaps with at least a part of the central elastic member when viewed in the thickness direction.

According to Aspect 13, in cases where the folded-back portion overlaps with the central elastic member to a greater extent, it is possible to reduce the risk that the central elastic members 17 and another pants-type absorbent article stored adjacent on the folded-back portion side adhere to each other. Furthermore, in cases where the folded-back portion overlap with the central elastic member to a lesser extent, it is possible to reduce the risk that the waist portion and the central elastic member adhere to each other due to adhesive that has seeped out as a result of compression.

Aspect 14 is a package of a pants-type absorbent article according to any one of Aspects 1 to 13, wherein: the pair of waist portions are joined by a pair of side joining portions at both side portions in the lateral direction; a plurality of waist elastic members that stretch/contract in the lateral direction are fixed to the pair of waist portions with an adhesive, being arranged side-by-side in the up-down direction; in an upper end portion, in the up-down direction, of the pair of waist portions, a region adjacent to a lateral inner end of the pair of side joining portions has a region where the adhesive that fixes the waist elastic members is not applied, or a low basis-weight region where a basis weight of the adhesive is lower than in a region located inside the low basis-weight region in the lateral direction; and in the packaged state, both end portions, in the lateral direction, of the pair of waist portions are folded back inward in the lateral direction.

According to Aspect 14, since the region adjacent to the inner end of the side joining portion has the region where no adhesive is present or the region where the basis weight of the adhesive is low, even if the both end portions of the waist portion that has been folded inward in the lateral direction are close to or overlap the central elastic member, it is less likely for the adhesive of the central elastic member and the adhesive of the waist elastic member to adhere to each other. This prevents tearing of the absorbent article and coming off of the elastic members.

Aspect 15 is a package of a pants-type absorbent article according to Aspect 14, wherein: the region adjacent to the lateral inner end of the pair of side joining portions has a region that extends an entire area overlapping the side joining portion in the up-down direction and where the adhesive that fixes the waist elastic members is not applied, or a low basis-weight region that extends the entire area overlapping the side joining portion in the up-down direction and where a basis weight of the adhesive is lower than in a region located inside the low basis-weight region in the lateral direction; and in the packaged state, the both end portions, in the lateral direction, of the pair of waist portions are folded back inward in the lateral direction.

According to Aspect 15, since, over the entire area overlapping the side joining portion in the up-down direction, the region adjacent to the inner end of the side joining portion has the region where the adhesive is not present or the region where the basis weight of the adhesive is low, even if the waist portion is folded back more deeply, it is less likely for the adhesive of the central elastic member and the adhesive of the waist elastic member to adhere to each other. This prevents tearing of the absorbent article and coming off of the elastic members.

Aspect 16 is a package of a pants-type absorbent article according to any one of Aspects 1 to 15, wherein: the pair of waist portions are joined by a pair of side joining portions at both side portions in the lateral direction; the pair of waist portions include an inner-layer sheet and an outer-layer sheet that is located on a non-skin side relative to the inner-layer sheet; at least one of the pair of waist portions has a sheet portion that is located farthest on the skin-facing side of that waist portion; the sheet portion is fixed to the inner-layer sheet with an adhesive; a portion where a region adjacent to a lateral inner end of the pair of side joining portions and the sheet portion overlap when viewed in the thickness direction has a region where the adhesive that fixes the sheet portion is not applied, or a low basis-weight region where a basis weight of the adhesive is lower than in a region located inside the low basis-weight region in the lateral direction; and in the packaged state, both end portions, in the lateral direction, of the pair of waist portions are folded back inward in the lateral direction.

According to Aspect 16, in the region adjacent to the inner end of the side joining portion, since the portion that overlaps the sheet portion has a region where no adhesive is present or a region where the basis weight of the adhesive is low, even if the both end portions of the waist portion that has been folded inward in the lateral direction are close to or overlap the central elastic member, it is less likely for the adhesive of the central elastic member and the adhesive of the sheet portion to adhere to each other.

Aspect 17 is a package of a pants-type absorbent article according to any one of Aspects 1 to 16, wherein the pants-type absorbent article includes an absorbent main body that has an absorbent core, and in the packaged state, a part of the absorbent core is bent.

According to Aspect 17, the absorbent core is a high-rigidity region, and therefore is a portion where the restoring force, which tends to return to its original position, is strong. The portion of the absorbent core that is bent in the packaged state attempts to return to its original shape by the restoring force even if compressed, thus suppressing the pressure on the central elastic region. Therefore, it reduces the likelihood of product defects in the portion of the central elastic member in the packaged state.

Aspect 18 is a package of a pants-type absorbent article according to any one of Aspects 1 to 17, wherein the pair of waist portions are provided with a plurality of waist elastic members that stretch/contract in the lateral direction, arranged side-by-side in the up-down direction, and a force required to stretch the waist elastic members from the natural state by a unit length in the lateral direction is greater than a force required to stretch the central elastic member from the natural state by the unit length in the up-down direction.

According to Aspect 18, since the force required to stretch the waist elastic members by a unit length is greater (i.e., the contraction force is larger), more folds generates in the waist portion when the waist elastic members contract. The presence of numerous folds can reduce compression even when the waist portion is folded and compressed in the packaged state, making it possible to prevent the waist portion from adhering to the crotch portion due to the adhesive that has seeped out.

### Embodiment

Hereinafter, an embodiment will be described by way of example, with reference to a package 100 for disposable diapers that stores multiple pant-type disposable diapers for adults, the package 100 being used as a package for pants-type absorbent articles according to the present invention (hereinafter also simply referred to as the "package 100"). However, the pants-type absorbent articles that are packed in the package 100 are not limited to pant-type disposable diapers for adults; for example, they may be pants-type disposable diapers for infants or shorts-type sanitary napkins.

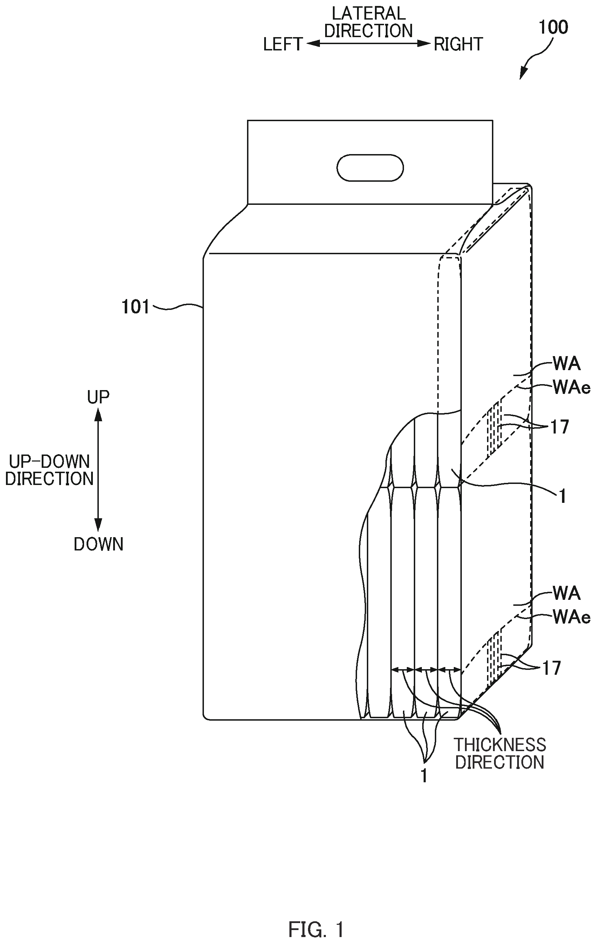

FIG. 1 is a schematic perspective view of the package 100 for disposable diapers. The package 100 includes a containment member 101, which is a bag-shaped member, and a plurality of pants-type absorbent articles 1 (pants-type disposable diapers 1, hereinafter also simply referred to as "diapers 1") packed inside the containment member 101. Furthermore, the package 100 (containment member 101) has an up-down direction and a lateral direction that intersect with each other. As shown in FIG. 1, in the package 100 in a state where diapers 1 are stored in the containment member 101 (hereinafter also referred to as "the packaged state"), a plurality of diapers 1 are stored in two stories, upper and lower, but the present invention is not limited thereto. Furthermore, the package 100 has a rectangular parallelepiped shape; however, it is not limited thereto and may have any shape.

Examples of materials forming the containment member 101 include thermoplastic resin films such as polyethylene (PE) and polypropylene (PP). Furthermore, the containment member 101 is not limited thereto, and at least a portion thereof may be formed of paper.

### Basic configuration of a pants-type disposable diaper 1

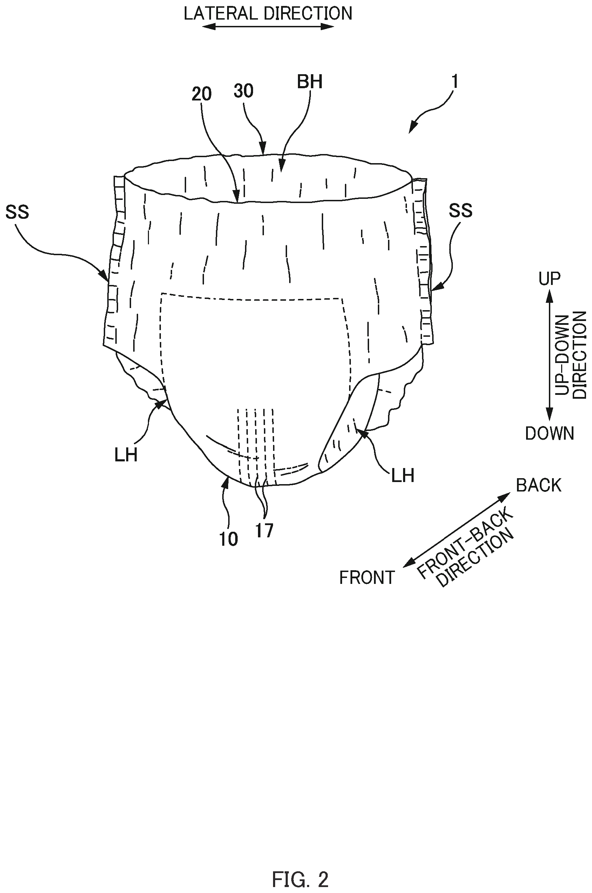

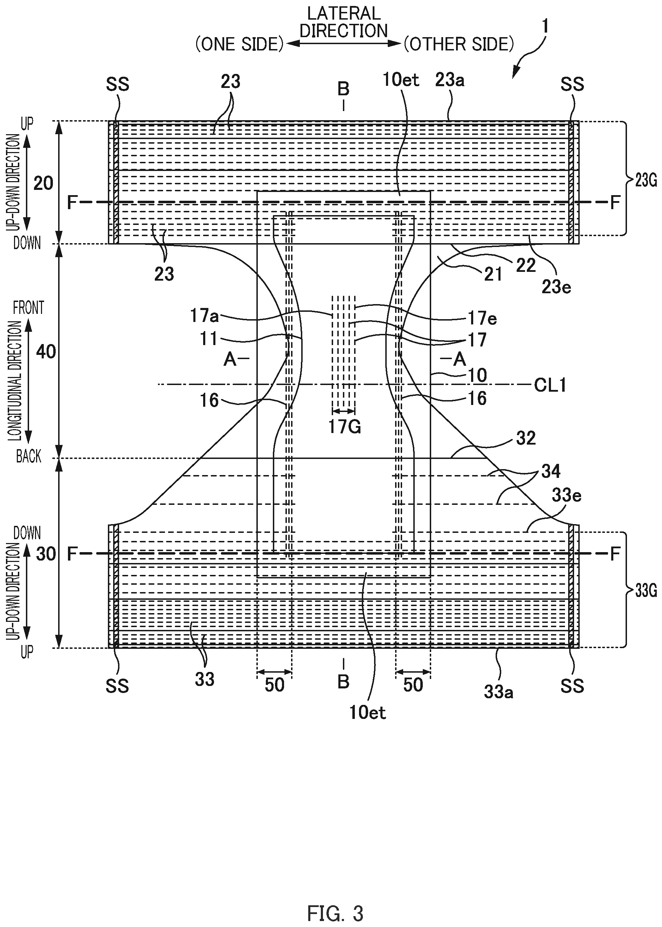

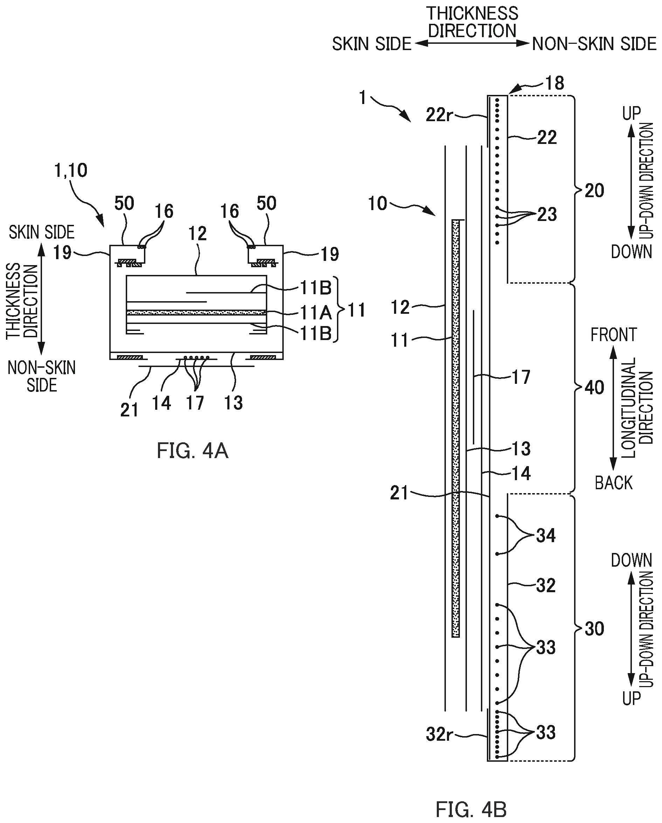

FIG. 2 is a schematic perspective view of the diaper 1 when viewed from the front side. FIG. 3 is a schematic plan view of the diaper 1 in an unfolded and stretched state, when viewed from the skin side of the wearer. FIG. 4A is a schematic cross-sectional view taken along line A-A in FIG. 3, and FIG. 4B is a schematic cross-sectional view taken along line B-B in FIG. 3.

The "unfolded state" of the diaper 1 refers to the state in which the diaper 1 in the pants-shaped state of FIG. 2 is separated into a front waist portion 20 (described later) and a back waist portion 30 (described later) by detaching the side joining portion SS (described later) on two sides in the lateral direction, and opening the diaper 1 in the up-down direction, thereby unfolding the diaper 1 on a flat surface. Furthermore, the "stretched state" of the diaper 1 refers to the state in which the product (diaper 1) is stretched without wrinkles, specifically, the state of being stretched to the extent that the dimensions of constituent members of the diaper 1 (e.g., an absorbent main body 10, the front waist portion 20, etc.) match or are close to the dimensions of the members on their own.

In the pants-shaped state as shown in FIG. 2 when worn, the diaper 1 has an up-down direction, a lateral direction, and a front-back direction that intersect each other, and has a waist opening BH and a pair of leg openings LH. In the up-down direction, the side of the waist opening BH is the upper side, and the side of the wearer's crotch region is the lower side. In the front-back direction, the wearer's abdominal side is the front side, and the dorsal side is the back side. Furthermore, in the unfolded and stretched state shown in FIG. 3, the diaper 1 has three mutually orthogonal directions: a longitudinal direction, a lateral direction, and a thickness direction (the direction that penetrate the paper plane in FIG. 3). Furthermore, the longitudinal direction conforms to the up-down direction mentioned above. Furthermore, the one side in the longitudinal direction corresponds to the front side, and the other side corresponds to the back side. Furthermore, the diaper 1 has the thickness direction in which materials are stacked, as shown in FIGS. 4A and 4B. In the thickness direction, the side that comes into contact with the wearer is the skin side and the opposite side is the non-skin side.

The diaper 1 has an absorbent main body 10 and an exterior body 18. The exterior body 18 includes a pair of waist portions 20 and 30, and a crotch portion 40 located between the wearer's legs. The exterior body is bonded to the non-skin-side surface of the absorbent main body 10 with an adhesive, or the like. Of the pair of waist portions 20 and 30, the one located on the wearer's front side is also referred to as the front waist portion 20, and the one located on the wearer's back side is also referred to as the back waist portion 30. The front waist portion 20 and the back waist portion 30 are joined by a pair of the side joining portions SS, at the both side portions in the lateral direction. Examples of a joining method for the side joining portions SS may include methods such as thermal welding, ultrasonic welding, and joining by using an adhesive.

The absorbent main body 10 includes an absorbent body 11, a liquid-permeable top sheet 12 located on the skin side relative to the absorbent body 11, a liquid-impermeable back sheet 13 located on the non-skin side relative to the absorbent body 11, and an exterior sheet 14, as shown in FIGS. 4A and 4B. The absorbent body 11 includes an absorbent core 11A that absorbs and retains excreted fluid such as urine, and a liquid-permeable core-wrapping sheet 11B that covers the absorbent core 11A. Examples of the absorbent core 11A include those formed by molding a liquid absorbent material such as pulp fibers and superabsorbent polymer (SAP) into a predetermined shape. On the other hand, examples of the core-wrapping sheet 11B include tissue paper, nonwoven fabric, and the like. Note that the absorbent core 11A does not need to be covered by the core-wrapping sheet 11B.

The top sheet 12 is formed of nonwoven fabric such as air-through nonwoven fabric, spunbond nonwoven fabric, or the like. The back sheet 13 is, for example, a liquid-impermeable and breathable film made of polyethylene or polypropylene. The exterior sheet 14 is formed, for example, of nonwoven fabric equivalent to the top sheet 12.

As shown in FIGS. 3 and 4A, the both lateral side portions of the absorbent main body 10 is provided with a pair of leak-proof wall portions 50, 50 extending along the longitudinal direction of the absorbent main body 10. In the present embodiment, the leak-proof wall portions 50, 50 can stand upright on the skin side, and are formed by providing, between a pair of side sheets 19, 19 located on the skin side of the top sheet 12, leak-proof-wall elastic members 16 (e.g., elastic strings) in a state of stretching along the longitudinal direction, but the present invention is not limited thereto. For example, the sheet members that constitutes the exterior of the absorbent main body 10 may be formed integrally with the absorbent main body 10 by being folded. However, in the diaper 1, the leak-proof wall portions 50,50 does not necessarily have to be provided.

Furthermore, in the absorbent main body 10, the crotch portion 40 has central elastic members 17 that can stretch/contract in the longitudinal direction (up-down direction), on the non-skin side in the thickness direction relative to the absorbent body 11 and at the central portion in the lateral direction. Specifically, in the present embodiment, the thread-like central elastic members 17 (e.g., elastic strings) are attached, with an adhesive, being sandwiched between the back sheet 13 and the exterior sheet 14. In this example, five central elastic members are provided at intervals in the lateral direction, but the number of the central elastic members is not limited thereto; it can be one or more. Furthermore, the central elastic members is not limited to thread-like material; for example, it may be a stretchable film, stretchable nonwoven fabric, or the like. Furthermore, FIG. 3 shows only portions of the central elastic members that exhibit stretchability (so-called effective length portions).

Furthermore, as shown in FIGS. 3, 4A and 4B, the central elastic members extend from the front side across the center line to the back side in the longitudinal direction, in the lateral central portion of the absorbent main body 10. However, the length, in the longitudinal direction, of the central elastic members 17 are not limited thereto; it is sufficient that they are provided at least in the crotch portion 40, which faces the wearer's crotch. As a result, in the crotch portion 40, the fit at the center, in the lateral direction, of the absorbent main body 10 improves.

As shown in FIGS. 4A and 4B, the exterior body 18 has an inner-layer sheet 21, a front outer-layer sheet 22, and a back outer-layer sheet 32.

The inner-layer sheet 21 is arranged extending from the front waist portion 20 through the crotch portion 40 to the back waist portion 30 (FIG. 4B). The width (length in the lateral direction) of the inner-layer sheet 21 in the front waist portion 20 and the back waist portion 30 is greater than the width (length in the lateral direction) of the inner-layer sheet 21 in the crotch portion 40 (FIG. 3). For the inner-layer sheet 21, it is possible to use air-through nonwoven fabric, spunbond nonwoven fabric, SMS nonwoven fabric (spunbond/meltblown/spunbond nonwoven fabric), a waterproof film, or the like.

As shown in FIGS. 3 and 4B, the front outer-layer sheet 22 is located on the non-skin side relative to the inner-layer sheet 21, and the front outer-layer sheet 22 and the inner-layer sheet 21 constitute the front waist portion 20. The outer end (upper end), in the longitudinal direction, of the front outer-layer sheet 22 is folded back towards the skin side. The folded-over portion 22r of the front outer-layer sheet 22 wraps the one end (outer end), in the longitudinal direction, of the inner-layer sheet 21. Furthermore, the outer end (upper end), in the longitudinal direction, of the front outer-layer sheet 22 does not have to be folded back.

Similarly, as shown in FIGS. 3 and 4, the back outer-layer sheet 32 is located on the non-skin side relative to the inner-layer sheet 21, and the back outer-layer sheet 32 and the inner-layer sheet 21 constitute the back waist portion 30. The outer end (upper end), in the longitudinal direction, of the back outer-layer sheet 32 is folded back towards the skin side. The folded-over portion 32r of the back outer-layer sheet 32 wraps the other end (outer end), in the longitudinal direction, of the inner-layer sheet 21. Furthermore, the outer end (upper end), in the longitudinal direction, of the back outer-layer sheet 32 does not have to be folded back.

For the front outer-layer sheet 22 and the back outer-layer sheet 32, it is possible to use air-through nonwoven fabric, spunbond nonwoven fabric, SMS nonwoven fabric (spunbond/meltblown/spunbond nonwoven fabric), or the like.

The front waist portion 20 and the back waist portion 30 each have waist elastic members 23 and 33 that can stretch/contract in the lateral direction. A plurality of the waist elastic members 23, 33 are arranged at intervals in the up-down direction between the inner-layer sheet 21 and the front outer-layer sheet 22, and between the inner-layer sheet 21 and the back outer-layer sheet 32, and the waist elastic members 23, 33 are joined and fixed with an adhesive such as hot-melt adhesive in a state of stretching in the lateral direction (see FIGS. 3 and 4B). Therefore, the front waist portion 20 and the back waist portion 30 stretch in the lateral direction to fit around the wearer's waist. In addition, in the present embodiment, elastic strings are used as the waist elastic members 23 and 33, the present invention is not limited thereto, and for example, a long, narrow stretchable sheet may also be used.

Furthermore, in the present embodiment, in the back waist portion 30, the back leg elastic members 34 are provided between the inner-layer sheet 21 and the back outer-layer sheet 32, and below the waist elastic members 33. The back leg elastic members 34 may, for example, be a plurality of elastic strings, or strip-shaped elastic sheet having stretchability. The back leg elastic members 34 are joined and fixed in the stretched state with an adhesive such as a hot-melt adhesive, and the back leg elastic members 34 make the diaper 1 easier to fit around the legs and buttocks on the back side.

The basic configuration of the diaper 1 is described above, but the above-described configuration of the diaper 1 is merely a example and the present invention is not limited thereto. For example, the diaper 1 of the present embodiment has a configuration including the exterior body 18 having three parts: the front waist portion 20, the back waist portion 30, and the crotch portion 40 connecting them. However, the present invention is not limited thereto, and for example, diapers may have a configuration in which the front waist portion 20 and the back waist portion 30 are made of separate members and in which the non-skin-side surface of the absorbent main body 10 is exposed in the crotch portion, or diapers may have an exterior member made of a single member that extends continuously from the front waist portion 20 to the back waist portion 30.

### Folding of diaper 1 and packaging method

As described above, in the package 100, a plurality of the diapers 1 (pants-type absorbent articles) are stored in a folded state inside the containment member 101. The following describes a method for folding the diaper 1 and a packaging method.

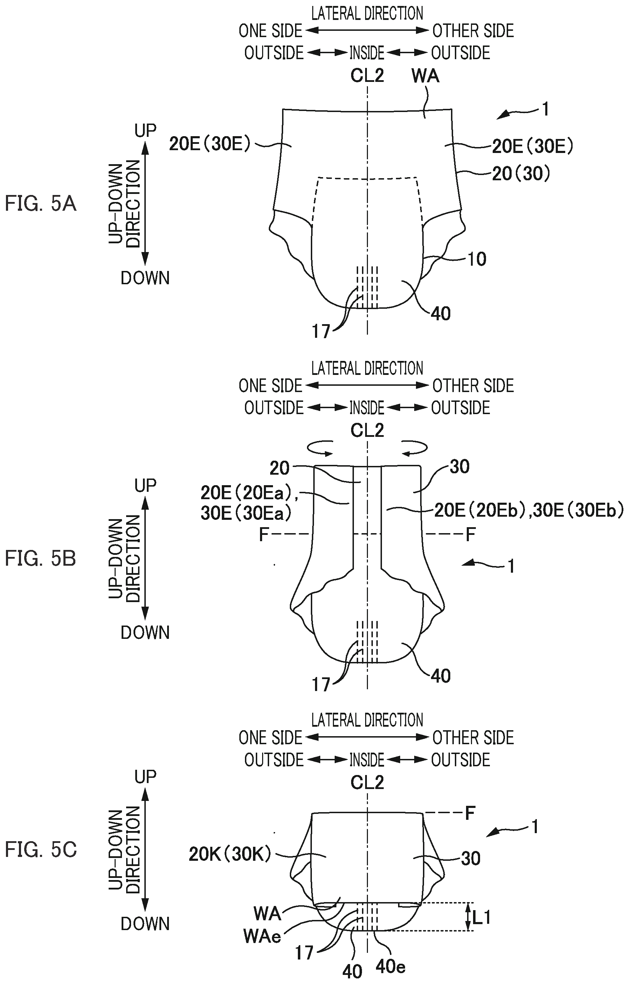

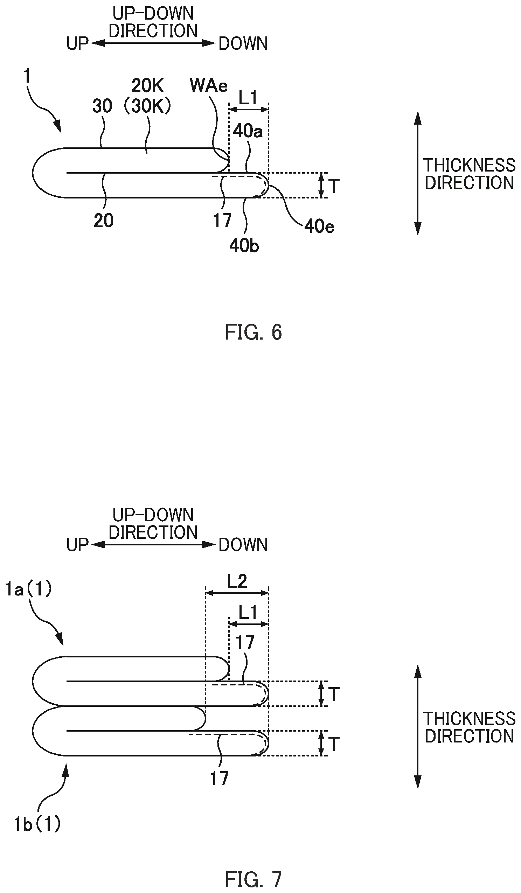

FIGS. 5A to 5C are diagrams illustrating how the diaper 1 is folded when stored. FIG. 6 is a schematic side view of the diaper 1 in a packaged state where the diaper 1 is packed in a containment member 101. When placing the diaper 1 into the containment member 101, first, from the natural state shown in FIG. 5A, the lateral one-side end portion 20Ea (30Ea) and the lateral other-side end portion 20Eb (30Eb) of the waist portion 20 (30) of the diaper 1 are folded inward in the lateral direction, as shown in FIG. 5B (hereinafter, "the one-side end portion 20Ea (30Ea) and the other-side end portion 20Eb (30Eb)" are also collectively referred to as "both end portions 20E (30E)").

Next, as shown in FIG. 5C, at the fold line F, the end portion WA, in the up-down direction, of the waist portion 20 (30) is folded back so as to be located on the crotch portion 40 side. In this way, the diaper 1 is folded into a compact shape as shown in FIG. 5C, and stored such that the up-down direction of the containment member 101 and the up-down direction of the diaper 1 are aligned, resulting in the packaged state as shown in FIG. 1. In the present embodiment, the both end portions 20E (30E), in the lateral direction, of the folded waist portions 20 (30), do not overlap in the thickness direction, but have a portion that overlaps with the central elastic members 17. However, the present invention is not limited thereto, and details of possible folding patterns will be described later.

Normally, when a pants-type absorbent article for adults such as the diaper 1 is stored in the containment member, it is often stored with a higher compressive force applied, in a state where the waist opening BH side folded towards the crotch side so as to make the absorbent article more compact, similar to the present embodiment. In such a packaged state, in cases where the central elastic members 17 exist in the crotch portion 40 as in the present embodiment, there is a risk that an adhesive for the central elastic members 17, or an adhesive for the waist elastic members arranged in the waist portions 20 (30) seeps out and adheres to adjacent products (diapers 1) within the containment member 101, causing the diaper 1 to tear or the central elastic members 17 to come off.

In this regard, in the package 100 of the present embodiment, in the packaged state where the tt diapers 1 are packed in the containment member 101, as described above, the end portion WA, in the up-down direction, of the waist portion 20 (30) of each diaper 1 is folded back towards the crotch portion 40, and a plurality of the diapers 1 are compressed in a state where they are stacked in the thickness direction of the diapers 1 (corresponding to the lateral direction of the package 100). Furthermore, in each diaper 1, the end WAe, in the up-down direction, of the folded waist portion 20 (30) does not extend beyond the end 40e, in the up-down direction, of the crotch portion 40 (see FIG. 5C). Furthermore, in the present embodiment, as shown in FIGS. 5C and 6, in at least one of the plurality of stored diapers 1, the distance L1 from the end WAe of the waist portion 20 (30) to the end 40e of the crotch portion 40 at the lateral center position CL2 (FIG. 5A) is longer than from the average thickness 1 of the crotch portion 40. In other words, when compressed and packed in the containment member 101, the distance from the end WAe of the folded waist portion 20 (30) to the end 40e of the crotch portion 40 is greater than the average thickness of the crotch portion 40.

In the packaged state, by separating the end WAe of the waist portion 20 (30) and the end 40e of the crotch portion 40, the separating portion becomes thinner compared to cases where the diaper 1 is folded such that the end WAe of the waist portion 20 (30) and the end 40e of the crotch portion 40 coincide or are close together. This weakens the compressive force applied to the separating portion, making it less likely for the diaper 1 to come into contact with another diaper 1 adjacent in the thickness direction. This makes it possible to better prevent the diapers 1 from sticking together (adhering) due to seeping out of the adhesive applied to the central elastic members 17, compared to cases where the end WAe and the end 40e are not separated.

The aforementioned "distance L1" can be measured by the following method. First, in the present embodiment, in the packaged state, a plurality of the diapers 1 are compressed in the thickness direction of the diapers 1, and the thickness direction corresponds to the lateral direction of the package 100. When taking measurements, the package 100 is clamped by the jigs from both sides in the lateral direction, and a portion of the containment member 101 is cut off from the portion of the contained diaper 1 where the end WAe of the waist portion 20 (30) and the crotch portion 40 can be identified. Furthermore, when clamping the package 100 with the jigs, the compressed state is maintained by clamping it so that the length, in the lateral direction, of the package 100 is approximately ±5% of the length of the package 100 in the compressed state. In the cut-off portion, the distance, at the lateral center position of the diaper 1, from the end WAe of the waist portion 20 (30) to the end 40e of the crotch portion 40 is measured, and this distance is defined as the distance L1. Furthermore, when the diapers 1 are packed in a state where the measurement location is hidden inside, while the package 100 is clamped with jigs, a portion of the containment member 101 and a portion of the diaper 1 are cut away until the measurement location becomes visible, and the measurement is then taken in that visible state.

Furthermore, the "average thickness T" of the crotch portion 40 is determined as follows. In the present embodiment, it is assumed that the thickness direction of the diaper 1 corresponds to the lateral direction of the package 100, the lateral length of the package 100 in the packaged state first is measured (a length W). In the package 100 (containment member 101), 10 diapers 1 are stored side-by-side in the lateral direction. From the length W, the thickness of the containment member 101 at two locations, namely the left end and right end in the lateral direction, is subtracted, and the resulting value is divided by the number of diapers, namely 10. In other words, it is {length W - (thickness of the containment member 101 × 2)}/10. The value obtained in this manner is taken as the "average thickness T".

FIG. 7 is a diagram illustrating adjacent diapers 1 in the packaged state. Generally, in the packaged state, adjacent diapers 1 overlap tightly without any gaps due to compression, but in FIG. 7, for convenience, the shapes of the diapers 1 are shown in a schematic manner. As shown in FIG. 7, among the diapers 1 that are stored, as described above, the diaper 1 that has a distance L1, at the center position CL2 of the lateral direction, from the end WAe of the waist portion 20 (30) to the end 40e of the crotch portion 40 is designated as a diaper 1a. Then, in the packaged state, another diaper 1 adjacent to the diaper 1a in the thickness direction is designated as a diaper 1b, and the distance, at the lateral center position of the diaper 1b, from the end WAe, in the up-down direction, of the folded-back waist portion 20 (30) to the end 40e, in the up-down direction, of the crotch portion 40 is defined as a length L2. In the present embodiment, it is preferable that the length L2 (distance L2) is different from the length L1 (distance L1) of the diaper 1 described above, and the length L2 is longer than at least half the length L1 or shorter than 1.5 times the length L1. Compared to cases where the positions of the ends WAe of the folded waist portions 20 (30) of the respective diapers 1 are aligned in the containment member 101, the pressure is distributed in cases where the positions of the ends WAe are different. Therefore, the adhesive that has seeped out makes it less likely for the crotch portion 40 where the central elastic members 17 are located and another diaper 1 (1b) to adhere to each other.

Furthermore, in the present embodiment, when the portion of the waist portion 20 (30) that is folded back towards the crotch portion 40 is designated as a folded-back portion 20K (30K) (see FIGS. 5C and 6), the folded-back portion 20K (30K) in the packaged state has a portion that overlaps with at least parts of the central elastic members 17 as viewed in the thickness direction. Furthermore, in cases where, for example, the folded-back portion 20K (30K) overlaps with the central elastic members 17 to a greater extent, it is possible to reduce the risk that the central elastic members 17 and another diaper 1 stored adjacent on the folded-back portion 20K (30K) side adhere to each other due to adhesive that has seeped out. On the other hand, in cases where the folded-back portion 20K (30K) overlap with the central elastic members 17 to a lesser extent, it is possible to reduce the risk that the waist portion 20 (30) and the central elastic members 17 adhere to each other due to adhesive that has seeped out as a result of compression.

Furthermore, the configuration of the folded-back portion 20K (30K) is not limited to having a portion that overlaps with at least parts of the central elastic members 17 as viewed in the thickness direction. A configuration is acceptable in which, in the packaged state, the upper end WAe of the folded-back portion 20K (30K) that is folded back towards the crotch portion 40 does not overlap with the central elastic members as viewed in the thickness direction. This makes it easier to avoid the adhesion between the waist portion 20 (30) and the central elastic members 17 in a compressed state due to adhesive that has seeped out.

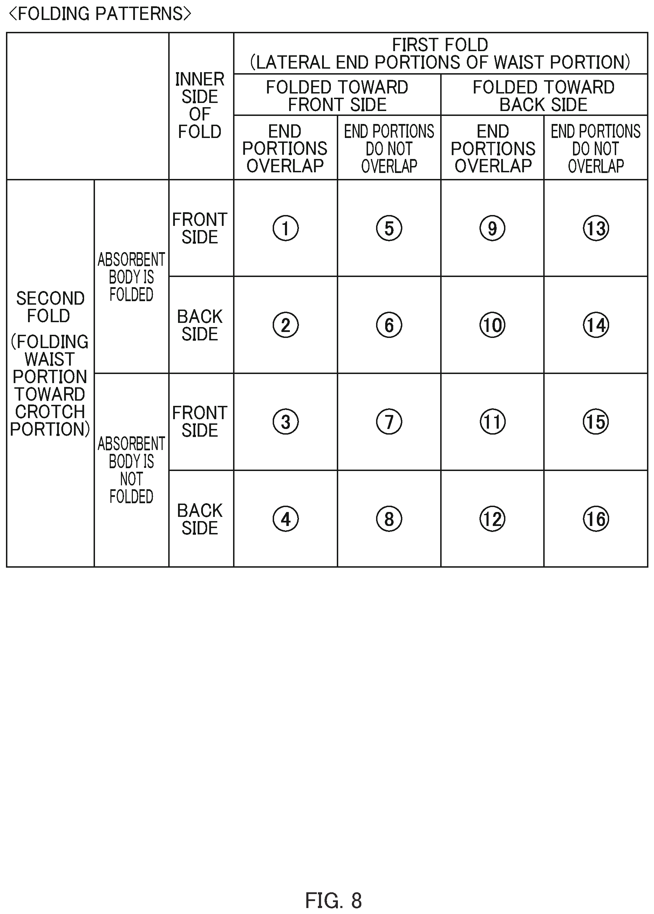

Here, the folding configuration when the diaper 1 is stored in the containment member 101 will be described in detail. FIG. 8 is a table showing the folding patterns of the diaper 1. First, when folding the diaper 1, as shown in FIG. 5B, the both lateral end portions (20Ea, 20Eb) of the waist portion 20 (30) of the diaper 1 are folded inward in the lateral direction. This folding is defined as a first fold. As shown in FIG. 8, the first fold includes two options: folding the both end portions (20Ea, 20Eb) towards the front side of the waist portion 20 (30) or folding the both end portions (20Ea, 20Eb) the back side. In the present embodiment, the folding is towards the front side.

In the first fold, in cases where both end portions (20ea, 20Eb) of the waist portion 20 (30) are folded to whichever of the front side or back side of the waist portion 20 (30), both end portions (20ea, 20Eb) of the waist portion 20 (30) are folded inward in the lateral direction, and therefore the portion that is folded inward becomes thicker. Furthermore, in the packaged state of the diaper 1, the thicker portion is more likely to come into contact with another diaper 1 adjacent in the containment member 101. Because the portion where the central elastic members 17 are located is thinner than the portion where the both end portions (20Ea, 20Eb) are folded back, even when a plurality of the diapers 1 are stacked on each other and compressed, the portion where the central elastic members 17 are located is less likely to come into contact with another diaper 1. This makes it possible to reduce the risk that the central elastic members 17 and another diaper 1 adhere to each other due to adhesive that has seeped out.

Next, the both end portions (20Ea, 20Eb) are folded towards either front side or back side, and this folding is made in such a manner that the both end portions (20Ea, 20Eb) overlap in the thickness direction, or that they do not overlap. In the present embodiment, as shown in FIG. 5B, the both end portions (20Ea, 20Eb) is folded in such a manner that they do not overlap.

After folding back the both lateral end portions (20Ea, 20Eb) of the waist portion 20 (30), the end portion WA, in the up-down direction, of the waist portion 20 (30) is folded back so as to be on the crotch portion 40 side, as shown in FIG. 5C. This folding is defined as a second fold. As shown in FIG. 8, the second fold includes two options: folding the absorbent body 11 together with it or not folding the absorbent body 11. In the present embodiment, the absorbent body 11 is not folded in the second fold. Furthermore, when folding back the end portion WA, in the up-down direction, of the waist portion 20 (30), there are two cases: folding with the front side of the waist portion 20 (30) inward, and folding with the back side inward.

In this way, by performing the first and second folds described above on the diaper 1, 16 folding configurations can be considered in total (see FIG. 8). The present embodiment corresponds to pattern 7 in FIG. 8, because the diaper 1 is folded toward the front side in the first fold, the both lateral end portions (20Ea, 20Eb) of the waist portion 20 (30) do not overlap with each other, and in the second fold, the absorbent body 11 is not folded and the front side is folded inward. Furthermore, in all cases when folded using the 16 folding configurations shown in FIG. 8, it is possible to reduce the risk that the waist portion 20 (30) and the central elastic members 17, or another adjacent diaper 1 and the central elastic members 17 adhere to each other in the packaged state due to adhesive that has seeped out as a result of compression. Some representative examples among these will be described below.

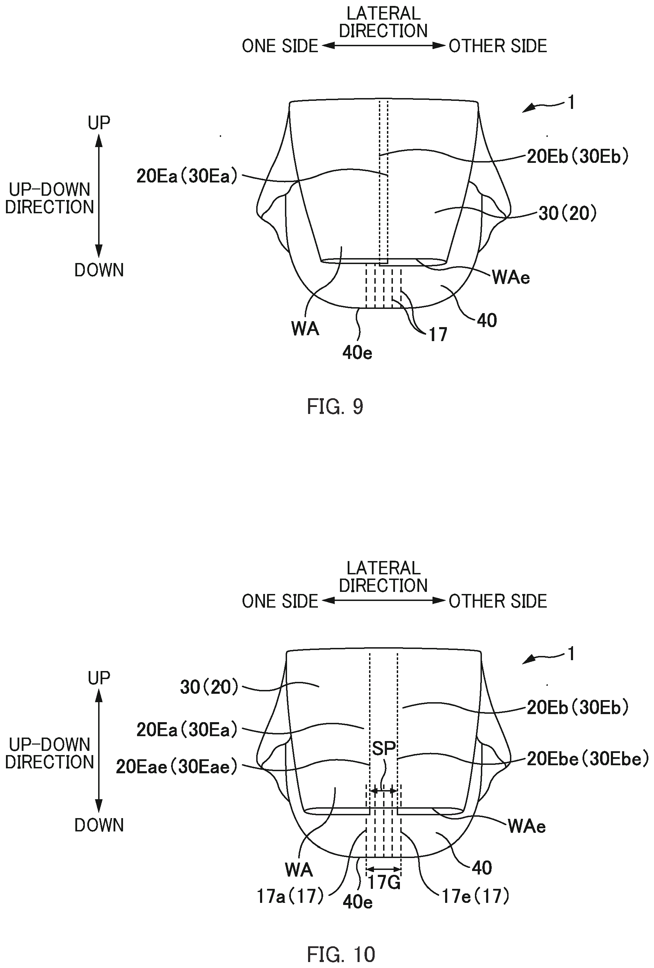

FIG. 9 is a diagram illustrating a modified example of a folded-back portion of the diaper 1 in the packaged state. As shown in FIG. 9, in the packaged state, the diaper 1 may be folded such that it has an overlapping portion where the lateral one-side end portion 20Ea (30Ea) and the lateral other-side end portion 20Eb (30Eb) of the folded waist portion 20 (30) overlap when viewed in the thickness direction (note that the thickness direction is the direction that penetrates the paper plane in FIG. 9). In the table in FIG. 8, patterns 1 to 4 and 9 to 12 are applicable. Furthermore, in the modified example shown in FIG. 9, the overlapping portion has a portion that overlaps the central elastic member 17 with respect to the lateral direction. The overlapping portion where the end portion 20Ea (30Ea) and the end portion 20Eb (30Eb) overlap when viewed in the thickness direction is a thicker portion. A difference in thickness occurs between the portion where this thicker portion (overlapping portion) and the central elastic members 17 overlap with respect to the lateral direction, due to the overlapping portion. The overlapping portion acts as a cushion, and, in the containment member 101, this makes it less likely for the central elastic members 17 to be susceptible to the effects of compression because the area of the central elastic members 17 overlaps such a cushion with respect to the lateral direction and is relatively thinner. Therefore, in the packaged state, it is possible to suppress seeping out of the adhesive of the central elastic members 17.

FIG. 10 is a diagram illustrating another modified example of the folded-back portion of the diaper 1 in the packaged state. In the diaper 1 shown in FIG. 10, the one-side end portion 20Ea (30Ea) and the other-side end portion 20Eb (30Eb) of the folded waist portion 20 (30) do not overlap when viewed in the thickness direction (the direction that penetrates the paper plane in FIG. 10) (in the table in FIG. 8, patterns 5 to 8 and 13 to 16 are applicable). Furthermore, in the lateral direction, when a region where the central elastic members 17 are located is defined as a central elastic region 17G, both ends 20Eae and 20Ebe (30Eae and 30Ebe) of the waist portion 20 (30) are located within the central elastic region 17G. Furthermore, the "central elastic region 17G" refers to the region between the central elastic member 17a that is located farthest on one side in the lateral direction and the central elastic member 17e that is located farthest on the other side in the lateral direction.

Since, in the packaged state, the lateral one-side end portion 20Ea and the lateral other-side end portion 20Eb of the folded waist portion 20 (30) do not overlap when viewed in the thickness direction, the waist portion 20 (30) is folded back with a gap SP in the lateral direction between the one-side end 20Eae and the other-side end 20Ebe of the waist portion 20 (30). Since, in the lateral direction, both ends (20Eae, 20Ebe) of the waist portion 20 (30) are located within the central elastic region 17G, the above-mentioned gap SP can suppress the pressure on the central elastic region 17G. More specifically, even if the both lateral end portions (20Ea, 20Eb) of the waist portion 20 (30) overlap the central elastic region 17G when viewed in the thickness direction (FIG. 10), in cases where there is a gap SP between the one-side end portion 20Eae and the other-side end portion 20Ebe, the pressure in the portion of the gap SP is weak in the compressed state. Therefore, in the portion of the gap SP, the adhesive that seeps out from the central elastic members 17 is less likely to transfer to the waist portion 20 (30).

On the other hand, in cases where the both lateral end portions (20Ea, 20Eb) of the waist portion 20 (30) do not overlap the central elastic region 17G when viewed in the thickness direction, and where both ends 20Eae, 20Ebe (30Eae, 30Ebe) of the waist portion 20 (30) are located, in the lateral direction, within the central elastic region 17G, the waist portion 20 (30) becomes thicker due to the laterally inward folding, and this makes the crotch portion 40 thinner compared to such a folded and thickened portion, thus reducing the pressure on the crotch portion 40. This makes it easier to prevent the crotch portion 40 and another diaper 1 from adhering to each other within the containment member 101 due to adhesive that has seeped out.

Furthermore, it is preferable that, within the containment member 101, there are a diaper 1 (first pants-type absorbent article) that has, in the packaged state, a portion where the lateral one-side end portion 20Ea and the lateral other-side end portion 20Eb of the folded waist portion 20 (30) overlap when viewed in the thickness direction as described above, and another diaper 1 (second pants-type absorbent article) in whic, in the packaged state, the lateral one-side end portion 20Ea and the lateral other-side end portion 20Eb of the folded waist portion 20 (30) do not overlap when viewed in the thickness direction. Since the size of the room that can accommodate multiple diapers 1 as the package 100 is fixed, the mixing of one diaper 1 (first pants-type absorbent article) with another diaper 1 (second pants-type absorbent article) distributes the areas of high pressure, making it less likely for adhesive to transfer as a whole.

Furthermore, as mentioned above, in the packaged state, the diaper 1 may be folded back at a position where the absorbent body 11 is folded. Specifically, when performing the second fold, the absorbent core 11A may be folded back so as to bend a portion thereof (in the table in FIG. 8, patterns 1, 2, 5, 6, 9, 10, 13, and 14). The absorbent core 11A is a high-rigidity region, and therefore is a portion where the restoring force, which tends to return to its original position, is strong. Of the absorbent core 11A, the portion that is bent in the packaged state attempts to return to its original shape by the restoring force even if compressed, thus reducing the pressure on the region where the central elastic members 17 are located. Therefore, it reduces the likelihood of product defects (tears or omissions) in the region where the central elastic members are located.

Furthermore, it is preferable that the above-mentioned average thickness T of the crotch portion 40 at the lateral center position CL2 (FIG. 5A) of the diaper 1 is 95% or less of the average thickness of the crotch portion 40 of the diaper 1 in the natural state where the diaper 1 is taken out of the containment member 101. Furthermore, more preferably, the average thickness is 85% or less of the average thickness of the crotch portion 40 of the diaper 1 in the natural state, and even more preferably 75% or less. In other words, by increasing the compression ratio, more diapers can be stored within the containment member 101. Furthermore, even if the compression ratio is increased and adjacent diapers 1 become in more close contact, by making the distance L1 from the upper end WAe of the waist portion 20 (30) to the end 40e of the crotch portion 40 longer than the average thickness T of the crotch portion 40 as in the present embodiment, it is possible to prevent the waist portion 20 (30) and the central elastic member 17 from adhering to each other due to the adhesive that has seeped out from the crotch portion 40 (central elastic members 17) of each diaper 1.

Furthermore, the central elastic members 17 in the present embodiment are thread-like ones. Because of the thread-like central elastic members, the amount of the adhesive for attaching the central elastic members 17 can be reduced compared to cases of applying adhesive to the entire surface of a thicker strip-shaped rubber or sheet-shaped stretchable material (stretchable film, stretchable nonwoven fabric, etc.) and then placing it. This makes it possible to further reduce the risk that the adhesive seeps out when compressed.

Returning to FIG. 3, the pair of waist portions 20, 30 are respectively provided with the plurality of waist elastic members 23, 33 that can stretch/contract in the lateral direction, located side-by-side in the up-down direction. Here, the region where the waist elastic members 23 (33) are arranged, that is, the region from the uppermost waist elastic member 23a (33a) to the lowermost waist elastic member 23e (33e) in the front waist portion 20 (back waist portion 30) is defined as a waist elastic region 23G (33G), and the region where the central elastic members 17 are arranged, that is, the region from the central elastic member 17a located farthest on the one side in the lateral direction to the central elastic member 17e located farthest on the other side in the crotch portion 40 is defined as the central elastic region 17G. Furthermore, in the present embodiment, the amount of an adhesive per unit area in the waist elastic region 23G (33G) is less than the amount of an adhesive per unit area in the central elastic region 17G. In the packaged state, the waist portions 20 and 30 are portions that become thicker by being folded. By reducing the amount of the adhesive in the waist elastic region 23G (33G) in such portions, it is possible to reduce the risk that the adhesive seeps out when compressed.

Furthermore, the amount of the adhesive per unit area in the waist elastic region 23G (33G) and the central elastic region 17G can be determined, for example, by the later-described solvent extraction method, in which the waist elastic members 23 (33) and the adhesive are separated from the inner-layer sheet 21 and the front outer-layer sheet 22 (back outer-layer sheet 32), the adhesive thus obtained is measured.

### Solvent extraction method of adhesive

First, in the stretched state, from the waist elastic region 23G (33G) and the central elastic region 17G, sheet materials (the inner-layer sheet 21 and the front outer-layer sheet 22 (the back outer-layer sheet 32)) and the waist elastic members 23 (33) are cut together into respective predetermined areas. Next, in a container such as a beaker, the sheet materials containing an adhesive and a solvent capable of dissolving the adhesive are mixed to obtain an adhesive solution in which the adhesive is dissolved in the solvent. Furthermore, the adhesive solution is collected from the container, and the solvent is removed from the adhesive solution, for example by drying the adhesive solution under reduced pressure using a rotary evaporator, thereby obtaining the adhesive. The amount of the adhesive thus obtained is taken as the amount of the adhesive per unit area. The solvent used to dissolve the adhesive should be selected appropriately depending on the type of adhesive, etc. If the adhesive to be measured is, for example, a hot-melt adhesive, then examples of solvents used to dissolve the adhesive include toluene, methyl ethyl ketone, and heptane.

Returning to FIG. 6, in the crotch portion 40 of the diaper 1 in the packaged state, when the side in the thickness direction where the folded waist portion 20 exists is defined as a fold-back inner side 40a, and the opposite side is defined as a fold-back outer side 40b, the length, in the up-down direction, of the central elastic member 17 is longer in on the fold-back inner side 40a than on the fold-back outer side 40b. Due to the shorter length of the central elastic member 17 on the fold-back outer side 40b, it is possible to reduce the risk that the central elastic member 17 and another diaper 1 adjacent on the fold-back outer side 40b within the containment member 101 adhere to each other.

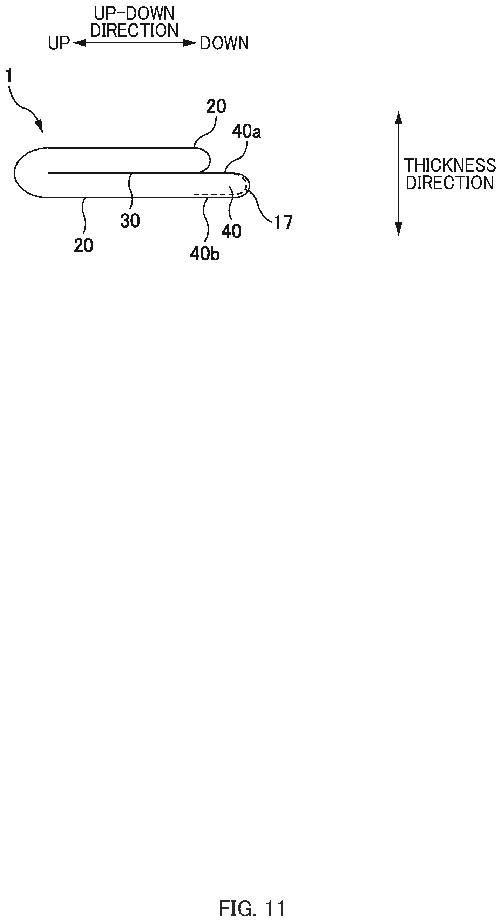

FIG. 11 is a diagram illustrating yet another modified example of the folded-back portion of the diaper 1. In the diaper 1 shown as the modified example of FIG. 11, when performing the second fold, the diaper 1 is folded back such that the inside of the fold is located on the back side (in the table in FIG. 8, patterns 2, 4, 6, 8, 10, 12, 14, and 16). Similar to the present embodiment, the central elastic member 17 extends in the longitudinal direction from the front side across the center line to the back side, at the lateral central portion of the absorbent main body 10. Specifically, of the length, in the longitudinal direction, of the central elastic member 17, the front length is longer than the back length. Therefore, in the crotch portion 40 of the diaper 1 in the packaged state in FIG. 11, when the side in the thickness direction where the folded waist portion 30 exists is defined as a fold-back inner side 40a, and the opposite side is defined as a fold-back outer side 40b, the length, in the up-down direction, of the central elastic member 17 is longer on the fold-back outer side 40b than on the fold-back inner side 40a. Due to the shorter length of the central elastic member 17 on the fold-back inner side 40a, it is possible to reduce the risk that the central elastic member 17 and another diaper 1 adjacent on the fold-back inner side 40a within the containment member 101 adhere to each other.

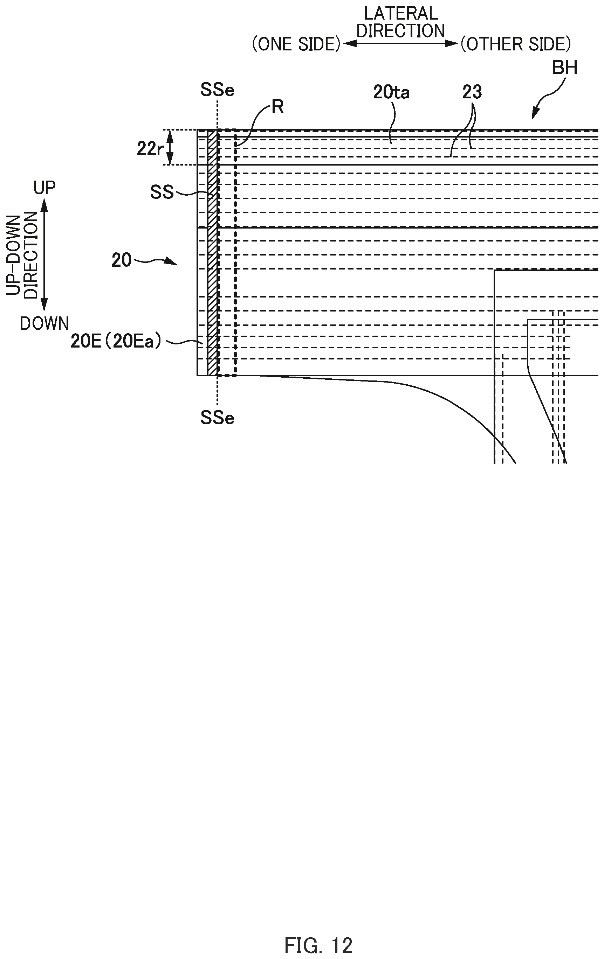

FIG. 12 is a partial enlarged view of the front waist portion 20 in the unfolded and stretched state. It should be noted that the front waist portion will be described in this example, but the back waist portion has a similar configuration. As described above, the pair of waist portions 20 and 30 of the diaper 1 are joined by the pair of side joining portions SS at the both side portions in the lateral direction. Furthermore, the plurality of waist elastic members 23 and 33 that stretch/contract in the lateral direction are fixed to the pair of waist portions 20 and 30 with an adhesive, being arranged side-by-side in the up-down direction. Then, as shown in FIG. 12, in the upper end portion 20ta of the front waist portion 20 in the up-down direction (that is, the portion of the waist opening BH), the region R adjacent to the lateral inner end SSe of the side joining portion SS is a region where the adhesive fixing the waist elastic members 23 is not applied. Furthermore, the region R is a region within the both lateral end portions 20E of the front waist portion 20, which is folded back inward in the lateral direction when stored. Even if the both end portions 20E that have been folded are close to or overlap the central elastic members 17 in the packaged state, the presence of the region R where an adhesive is not applied can reduce the adhesion between the adhesive of the central elastic members 17 and the adhesive of the waist elastic members 23 that have seeped out. This prevents tearing of the diaper 1 and coming off of the elastic members.

Furthermore, the above-described region R adjacent to the lateral inner end SSe of the side joining portion SS is not limited to a region where the adhesive that fixes the waist elastic members 23 is not applied, and the region R may also have a low basis-weight region in which the basis weight of the adhesive is lower than a region located inside it in the lateral direction. Since the region R has the low basis-weight region, which has a lower basis weight of the adhesive, even if the folded both end portions 20E are similarly close to or overlap the central elastic members 17 in the packaged state, it is possible to prevent the adhesion between the adhesive of the central elastic members 17 and the adhesive of the waist elastic members 23.

Furthermore, as shown in FIG. 12, the region R adjacent to the lateral inner end SSe of the side joining portion SS is not limited to the upper end portion 20ta, in the up-down direction, of the front waist portion 20, but is the region that extends over the entire area overlapping the side joining portion SS in the up-down direction and where the adhesive fixing the waist elastic members 23 is not applied. Furthermore, the present invention is not limited thereto. For example, the region R may have a low basis-weight region that extends over the entire area overlapping the side joining portion SS in the up-down direction and in which the basis weight of the adhesive is lower than a region located inside the lateral direction. In the packaged state, the entire area overlapping the side joining portion SS in the up-down direction is included in the both lateral end portions 20E of the front waist portion 20, which are folded back inward in the lateral direction. Therefore, even if the folded both end portions 20E are close to or overlap the central elastic members 17 in the packaged state, since the region R has the region where the adhesive is not applied or the low basis-weight region where the basis weight of the adhesive is low, it is possible to suppress adhesion between the adhesive of the central elastic members 17 and the adhesive of the waist elastic members 23 even when the waist portion 20 is folded back more deeply in the second fold. This prevents tearing of the diaper 1 and coming off of the elastic members.

Furthermore, in the present embodiment, the front waist portion 20 has the inner-layer sheet 21 and the front outer-layer sheet 22 (outer-layer sheet) that is located on the non-skin side relative to the inner-layer sheet 21, and the back waist portion 30 has the inner-layer sheet 21 and the back outer-layer sheet 32 (outer-layer sheet) that is located on the non-skin side relative to the inner-layer sheet 21. Furthermore, at least one of such a pair of waist portions 20 and 30 may have a sheet portion located farthest on the skin-facing side. In the present embodiment, the folded-over portion 22r (32r) of the front outer-layer sheet 22 (back outer-layer sheet 32) is the sheet portion. The sheet portion (folded-over portion 22r (32r)) is fixed to the inner-layer sheet 21 with an adhesive. Furthermore, within the region R adjacent to the lateral inner end SSe of the side joining portion SS shown in FIG. 12, the portion that overlaps the sheet portion (folded-over portion 22r (32r)) when viewed in the thickness direction is a region where no adhesive fixing the sheet portion (folded-over portion 22r (32r)) is applied. Furthermore, it is not limited to having the region where no adhesive for fixing is applied; the portion of the region R that overlaps the sheet portion (folded-over portion 22r (32r)) when viewed in the thickness direction may have a low basis-weight region in which the basis weight of the adhesive is lower than a region located inside it in the lateral direction. The region R is a region within the both lateral end portions 20E of the front waist portion 20, which is folded back inward in the lateral direction when stored. Therefore, even if the the both end portions 20E that have been folded are close to or overlap the central elastic members 17 in the packaged state, the presence of the region R where no adhesive is applied (or the region R having the low basis-weight region) can reduce the adhesion between the adhesive of the central elastic members 17 and the adhesive of the sheet portion (folded-over portion 22r (32r)).

Furthermore, the aforementioned sheet portion is not limited to the folded-over portion 22r (32r), but may be, for example, another sheet member which is different from the inner-layer sheet 21 and the front outer-layer sheet 22 (the back outer-layer sheet 32). As an example, a cover sheet (not shown) that is arranged to straddle the longitudinal end 10et of the absorbent main body 10 (see FIG. 3), and that is fixed to the inner-layer sheet 21 (and the absorbent main body 10) with an adhesive. Furthermore, within the region R adjacent to the lateral inner end SSe of the side joining portion SS shown in FIG. 12, it is preferable that the portion that overlaps the sheet portion (cover sheet) when viewed in the thickness direction has either a region where no adhesive fixing the sheet portion (cover sheet) is applied, or a low basis-weight region in which the basis weight of the adhesive is lower than a region located inside it in the lateral direction. Similarly, even if the both end portions 20E that have been folded are close to or overlap the central elastic members 17 in the packaged state, the presence of the region R where no adhesive is applied (or the region R having the low basis-weight region) can reduce the adhesion between the adhesive the central elastic members 17 and the adhesive of the sheet portion (cover sheet).

Furthermore, in the present embodiment, the pair of waist portions 20, 30 are respectively provided with the plurality of waist elastic members 23, 33 that can stretch/contract in the lateral direction, located side-by-side in the up-down direction. It is preferable that the force required to stretch the waist elastic members 23 and 33 from the natural state by a unit length in the lateral direction is greater than the force required to stretch the central elastic member 17 from the natural state by a unit length in the up-down direction. Because the force required to stretch the waist elastic members 23, 33 by a unit length is greater (i.e., the contraction force is larger), more folds generates in the waist portions 20, 30 when the waist elastic members 23, 33 contract. The presence of numerous folds can reduce compression even in the packaged state, where the waist portions 20 and 30 are folded and compressed, making it possible to suppress the waist portions 20 and 30 from adhering to the crotch portion 40 due to the adhesive that has seeped out.

Furthermore, a comparison between the force required to stretch the waist elastic members 23, 33 in the lateral direction from the natural state by a unit length and the force required to stretch the central elastic members in the up-down direction from the natural state by a unit length can be made by the following method.

First, in the stretched state, the waist elastic members 23 (33) are cut to a predetermined length and taken out of the waist portion 20 (30), and the central elastic members 17 are cut to a predetermined length and taken out of the crotch portion 40. In cases where the elastic member is in sheet form, the elastic members are cut to a predetermined width (e.g., a width of 10 mm). In cases where both are in thread form, for example, one thread is marked at a fixed length (e.g., 100 mm) in the stretch/contract direction of the elastic members and is cut out so as to be include that marked portion.

Next, the taken waist elastic members 23 (33) and central elastic members 17 are fixed by adjusting the initial spacing of a pair of chucks, which are spaced apart in the tensile direction of a tensile tester, to match the length of the sample (to a length that prevents the sample from sagging, or allows the sample to sag only slightly), and then fixing them so that the marked positions are placed at the inner end portions of the chucks (the width by which the chucks holds the sample being kept constant). It is preferable to compare values obtained by measuring the load when the sample is stretched by a unit length (e.g., 70% of the marking length) between the chucks, from a state where the both end portions (marked portions) of the sample are fixed by the chucks. In cases where the sample includes a plurality of thread-like elastic members, it is preferable to compare values converted to values per one thread-like elastic member after measurement.

### Others

The above embodiment is provided to facilitate understanding of the present invention and is not intended to limit its interpretation. The present invention may be modified or improved without departing from its spirit, and it goes without saying that the present invention includes equivalents thereof.
1 pants-type disposable diapers (pants-type absorbent article)
1a pants-type disposable diapers,
1b pants-type disposable diapers
10. absorbent main body
11 absorbent body
11A absorbent core
11B core-wrapping sheet
12 top sheet
13 back sheet
14 exterior sheet
16 elastic member
17, 17a, 17e central elastic member
17G central elastic region
18 exterior body
19 side sheet
20 waist portion (front waist portion)
20E both lateral end portions
20Ea one-side end portion
20Eae one-side end
20Eb other-side end portion
20Ebe other-side end
20K folded-back portion
20ta upper end portion
21 inner-layer sheet
22. front outer-layer sheet
22r folded-over portion
23, 23a, 23e waist elastic member
23G waist elastic region
30 waist portion (back waist portion)
30E both end portions
30Ea one-side end portion
30Eae one-side end
30Eb other-side end portion
30Ebe other-side end
30K folded-back portion
3. back outer-layer sheet
32r folded-over portion
33, 33a, 33e waist elastic member
33G waist elastic region
34 back leg elastic member
40 crotch portion
40a fold-back inner side
40b fold-back outer side
40e end in the up-down direction
50. leak-proof wall portion
100 package (package for pants-type absorbent articles)
101 containment member

## Claims

1. A package for pants-type absorbent articles comprising:
a plurality of pants-type absorbent articles; and
a containment member that stores the plurality of pants-type absorbent articles,
the pants-type absorbent article having an up-down direction, a lateral direction, and a thickness direction that are orthogonal to one another;
the pants-type absorbent article including:
a pair of waist portions;
a crotch portion that is located in a wearer's crotch; and
a central elastic member that is attached to a central portion, in the lateral direction, of the crotch portion with an adhesive, and can stretch/contract in the up-down direction,
in a packaged state in which the pants-type absorbent article is stored in the containment member,
an end portion, in the up-down direction, of the waist portion being folded back towards the crotch portion,
an end, in the up-down direction, of the folded waist portion not extending beyond an end, in the up-down direction, of the crotch portion,
the plurality of pants-type absorbent articles being compressed in a state of being stacked in the thickness direction, and
in at least one of the plurality of pants-type absorbent articles, a distance, at a lateral center position, from the end of the waist portion to the end of the crotch portion being longer than an average thickness of the crotch portion.

2. The package for pants-type absorbent articles according to claim 1, wherein
letting the distance be a length L1, and
letting a distance, at a lateral center position of another pants-type absorbent article that is adjacent in the thickness direction to the pants-type absorbent article in the packaged state, from the end, in the up-down direction, of the folded waist portion to the end, in the up-down direction, of the crotch portion be a length L2,
the length L2 is different from the length L1, and
the length L2 is longer than at least half the length L1 or is shorter than 1.5 times the length L1.

3. The package for pants-type absorbent articles according to claim 1 or 2, wherein
the average thickness is 95% or less of an average thickness of the crotch portion of the pants-type absorbent article in a natural state where the pants-type absorbent article is taken out of the containment member.

4. The package for pants-type absorbent articles according to claim 1 or 2, wherein
the central elastic member is a thread-like one.

5. The package for pants-type absorbent articles according to claim 1 or 2, wherein
the pair of waist portions are provided with a plurality of waist elastic members that stretch/contract in the lateral direction, arranged side-by-side in the up-down direction, and
an amount of an adhesive per unit area of a waist elastic region where the waist elastic members are arranged is less than an amount of an adhesive per unit area of a central elastic region where the central elastic member is arranged.

6. The package for pants-type absorbent articles according to claim 1 or 2, wherein
in the packaged state, an upper end of the waist portion that is folded back towards the crotch portion does not overlap with the central elastic member when viewed in the thickness direction.

7. The package for pants-type absorbent articles according to claim 1 or 2, wherein
in the packaged state, both lateral end portions of the waist portion are folded back inward in the lateral direction.

8. The package for pants-type absorbent articles according to claim 7, wherein
in the packaged state, the pants-type absorbent article has an overlapping portion where the lateral one-side end portion and the lateral other-side end portion of the folded waist portion overlap when viewed in the thickness direction, and
the overlapping portion has a portion that overlaps the central elastic member with respect to the lateral direction.

9. The package for pants-type absorbent articles according to claim 7, wherein
in the packaged state,
the lateral one-side end portion and the lateral other-side end portion of the folded waist portion do not overlap when viewed in the thickness direction, and
in the lateral direction, when a region where the central elastic member is located is defined as a central elastic region, both ends of the waist portion are located within the central elastic region.

10. The package for pants-type absorbent articles according to claim 7, wherein
within the containment member, there are:
a first pants-type absorbent article that has, in the packaged state, a portion where the lateral one-side end portion and the lateral other-side end portion of the folded waist portion overlap when viewed in the thickness direction; and
a second pants-type absorbent article in which, in the packaged state, the lateral one-side end portion and the lateral other-side end portion of the folded waist portion do not overlap when viewed in the thickness direction.

11. The package for pants-type absorbent articles according to claim 1 or 2, wherein
in the crotch portion in the packaged state, when a side in the thickness direction where the folded waist portion exists is defined as a fold-back inner side, and the opposite side is defined as a fold-back outer side,
a length, in the up-down direction, of the central elastic member is longer on the fold-back inner side than on the fold-back outer side.

12. The package for pants-type absorbent articles according to claim 1 or 2, wherein
in the crotch portion in the packaged state, when a side in the thickness direction where the folded waist portion exists is defined as a fold-back inner side, and the opposite side is defined as a fold-back outer side,
a length, in the up-down direction, of the central elastic member is longer on the fold-back outer side than on the fold-back inner side.

13. The package for pants-type absorbent articles according to claim 1 or 2, wherein
in the packaged state,
when a portion of the waist portion that is folded back towards the crotch portion is defined as a folded-back portion,
the folded-back portion has a portion that overlaps with at least a part of the central elastic member when viewed in the thickness direction.

14. The package for pants-type absorbent articles according to claim 1 or 2, wherein:
the pair of waist portions are joined by a pair of side joining portions at both side portions in the lateral direction;
a plurality of waist elastic members that stretch/contract in the lateral direction are fixed to the pair of waist portions with an adhesive, being arranged side-by-side in the up-down direction;
in an upper end portion, in the up-down direction, of the pair of waist portions, a region adjacent to a lateral inner end of the pair of side joining portions has
a region where the adhesive that fixes the waist elastic members is not applied, or
a low basis-weight region where a basis weight of the adhesive is lower than in a region located inside the low basis-weight region in the lateral direction; and
in the packaged state, both end portions, in the lateral direction, of the pair of waist portions are folded back inward in the lateral direction.

15. The package for pants-type absorbent articles according to claim 14, wherein:
the region adjacent to the lateral inner end of the pair of side joining portions has
a region that extends an entire area overlapping the side joining portion in the up-down direction and where the adhesive that fixes the waist elastic members is not applied, or
a low basis-weight region that extends the entire area overlapping the side joining portion in the up-down direction and where a basis weight of the adhesive is lower than in a region located inside the low basis-weight region in the lateral direction; and
in the packaged state, the both end portions, in the lateral direction, of the pair of waist portions are folded back inward in the lateral direction.

16. The package for pants-type absorbent articles according to claim 1 or 2, wherein:
the pair of waist portions are joined by a pair of side joining portions at both side portions in the lateral direction;
the pair of waist portions include an inner-layer sheet and an outer-layer sheet that is located on a non-skin side relative to the inner-layer sheet;
at least one of the pair of waist portions has a sheet portion that is located farthest on the skin-facing side of that waist portion;
the sheet portion is fixed to the inner-layer sheet with an adhesive;
a portion where a region adjacent to a lateral inner end of the pair of side joining portions and the sheet portion overlap when viewed in the thickness direction has
a region where the adhesive that fixes the sheet portion is not applied, or
a low basis-weight region where a basis weight of the adhesive is lower than in a region located inside the low basis-weight region in the lateral direction; and
in the packaged state, both end portions, in the lateral direction, of the pair of waist portions are folded back inward in the lateral direction.

17. The package for pants-type absorbent articles according to claim 1 or 2, wherein
the pants-type absorbent article includes an absorbent main body that has an absorbent core, and
in the packaged state, a part of the absorbent core is bent.

18. The package for pants-type absorbent articles according to claim 1 or 2, wherein
the pair of waist portions are provided with a plurality of waist elastic members that stretch/contract in the lateral direction, arranged side-by-side in the up-down direction, and
a force required to stretch the waist elastic members from the natural state by a unit length in the lateral direction is greater than a force required to stretch the central elastic member from the natural state by the unit length in the up-down direction.
